# EUROPEAN PATENT SPECIFICATION

(11) **EP 4 141 443 B1**
(45) Date of publication and mention of the grant of the patent: **07.05.2025**
(21) Application number: 21193187.8
(22) Date of filing: 26.08.2021
(51) Int. Cl.: G01N 33/487

(54) **APPARATUS, METHODS AND COMPUTER PROGRAMS FOR IDENTIFYING CHARACTERISTICS OF BIOLOGICAL SAMPLES**
VORRICHTUNG, VERFAHREN UND COMPUTERPROGRAMME ZUR IDENTIFIZIERUNG VON EIGENSCHAFTEN BIOLOGISCHER PROBEN
APPAREIL, PROCÉDÉS ET PROGRAMMES INFORMATIQUES PERMETTANT D'IDENTIFIER DES CARACTÉRISTIQUES D'ÉCHANTILLONS BIOLOGIQUES

(43) Date of publication of application: 01.03.2023
(73) Proprietor: Nokia Technologies Oy, 02610 Espoo (FI)
(72) Inventor: ZHENG, Mingde, Basking Ridge, NJ 08807 (US)
(74) Representative: Swindell & Pearson Limited

(56) References cited:
- WO-A1-2010/006253
- US-A1- 2015 104 821
- US-A1- 2021 007 638
- DATABASE WPI Week 200851, Derwent World Patents Index; AN 2008-H97014, XP002805496

## Description

### TECHNOLOGICAL FIELD

Examples of the disclosure relate to apparatus, methods and computer programs for identifying characteristics of biological samples. Some relate to apparatus, methods and computer programs for identifying characteristics of biological samples in-vivo.

### BACKGROUND

Analysis of biological samples can provide useful information about the biological sample. The information that is obtained can be used for controlling an electronic device, perform diagnosis, health monitoring or any other suitable purpose.

US 2015/104821 A1 (D1) relates to a method and a device for creating digital copies of the ionic-electric dynamics of cells or cell compartments, such as organisms, or an at least partly identifying dataset that allows sorting decisions in vitro and in vivo. According to D1, an object under test, for example cells, cell aggregates or whole organisms becomes stimulated by a physical or chemical stimulus whose dynamic characteristics can be controlled. A control unit analyzes the measurements and generates, based on their results, subsequent stimuli with well-defined characteristics.

### BRIEF SUMMARY

The invention is set out in the appended set of claims.

### BRIEF DESCRIPTION

Some examples will now be described with reference to the accompanying drawings in which:
FIG. 1 shows an example apparatus;
FIGS. 2A and 2B show another example apparatus and a biological sample;
FIG. 3 shows an example method;
FIG. 4 shows an example implementation of examples of the disclosure;
FIGS. 5A and 5B show example results;
FIGS. 6A and 6B show example plots obtained from electrical output signals;
FIGS. 7A and 7B show example plots obtained from electrical output signals;
FIG. 8 shows a plot obtained from electrical output signals;
FIG. 9 shows example plots obtained from electrical output signals;
FIG. 10 shows example plots obtained from electrical output signals;
FIGS. 11A to 11H show example electrical input and output pairings;
FIG. 12 shows example plots obtained from electrical output signals;
FIG. 13A to 13D show example plots obtained from electrical output signals; and
FIG. 14 shows an example apparatus.

### DETAILED DESCRIPTION

Examples of the disclosure relate to systems and methods for identifying characteristics of biological samples. The identified characteristics can be used for human-machine interactive purposes, health monitoring purposes, to provide control signals to electronic devices or computer programs, for diagnostic purposes or for any other suitable use.

In examples of the disclosure a plurality of electrical input signals can be provided to a biological sample so as to provide a plurality of corresponding electrical output signals. As the electrical output electrical signals have passed through the biological sample, the electrical output signals comprise of information about the properties of the biological sample. In examples of the disclosure, the electrical input signal can be controlled so that the electrical output signals comprise of general features and sub-features that enable characteristics of the biological sample to be identified. The use of these general features and sub-features can enable the characteristics to be identified without creating a reconstruction of the biological sample.

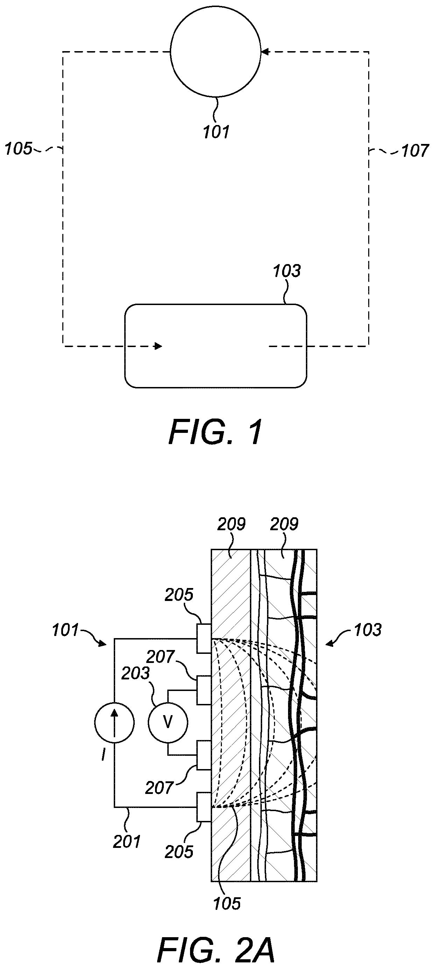

Fig. 1 schematically shows an example apparatus 101 for analysing a biological sample 103. The apparatus 101 could be used for electrical impedance tomography or other types of analysis of biological samples.

The biological sample 103 can comprise an in-vivo sample. The biological sample 103 can comprise any biological cellular matter. In some examples, the biological sample 103 could comprise a part of a user's body or any other suitable biological sample 103.

For instance, in some examples the biological sample 103 could comprise part of a person's arm and the apparatus 101 could be configured to detect movement or other changes of the arm.

The apparatus 101 can be configured to enable one or more characteristics of the biological sample 103 to be identified. The characteristic could comprise an intrinsic property of the biological sample 103. For example, the characteristics could comprise an indication about the types of structures or component within the biological sample 103. In such cases the characteristics could comprise information about a type of cell or tissue within the biological sample 103. The characteristics could comprise information relating to the position of such cells and tissues and/or the size, shape and/or conductivities of such cells and tissues or any other suitable information.

In some examples the characteristics could comprise changes within the biological sample 103. For example, the characteristics could comprise movement of the biological sample 103 and/or parts of the biological sample 103. In some examples the changes could comprise a change in size and/or shape and/or position of cells or tissues or other components within the biological sample 103. Other types of characteristics could be used in other examples of the disclosure.

The apparatus 101 comprises means for providing an electrical input signal 105 to the biological sample 103. The electrical input signal 105 can comprise any electrical signal that can stimulate the biological sample 103 so as to elicit one or more electrical properties of the biological sample 103. The electrical input signal 105 can be configured so that it does not cause irreversible damage to the cells and other components within the biological sample 103 or does not kill the cells or other components within the biological sample 103.

The electrical properties of the biological sample 103 that are elicited can be any properties that can be measured or detected by using the plurality of electrical input signals 105. The electrical properties that are elicited could comprise any one or more of resistance, capacitance, impedance, dielectric, phase or any other suitable property. The electrical properties of the biological sample 103 that are elicited could comprise properties of cells/tissues, or parts of cells and tissues, within the biological sample 103.

The apparatus 101 can be configured to provide the electrical input signal 105 in programmed waveforms. The timing, intensity, frequency and any other suitable property of the electrical input signal 105 can be controlled through the apparatus 101. The apparatus 101 can be configured to control the electrical input signal 105 so as to control one or more characteristics of the electrical output signals 107. The apparatus 101 can be configured to control the electrical input signal 105 so that the electrical output signal 107 can be used to identify the characteristics of the biological sample 103.

The apparatus 101 can comprise means for enabling the electrical input signals 105 to be provided to the biological sample 103. The means can comprise one or more electrodes configured so that the electrical input signals 105 can be applied to the biological samples 103. The electrodes can be configured to enable current from the electrodes to flow into the biological sample 103.

The apparatus 101 also comprises means for receiving electrical output signals 107 from the biological sample 103. The electrical output signals 107 correspond to the electrical input signals 105 in that they can be provided by the electrical input signals 105 passing through the biological sample 103. As the electrical output signals 107 comprise signals that have passed through the biological sample 103, the properties of the electrical output signals 107 are determined by the electrical properties of the biological sample 103. This enables the electrical output signals 107 to provide information relating to the electrical properties of the cells within the biological sample 103 and so can provide information relating to one or more characteristics of the biological sample 103.

The apparatus 101 also comprises means for enabling analysis of the electrical output signals 107. In some examples the electrical output signals 107 can be analysed to determine one or more general features and one or more sub-features within the plurality of electrical output signals 107. The one or more general features and one or more sub-features can enable one or more characteristics of the biological sample 103 to be identified. In some examples the general features or sub-features can be analysed by comparing them to reference signals. This can enable particular types of characteristics to be identified and/or monitored.

Fig. 2A schematically shows another example apparatus 101 and biological sample 103. In this example the biological sample 103 comprises a part of a user's body.

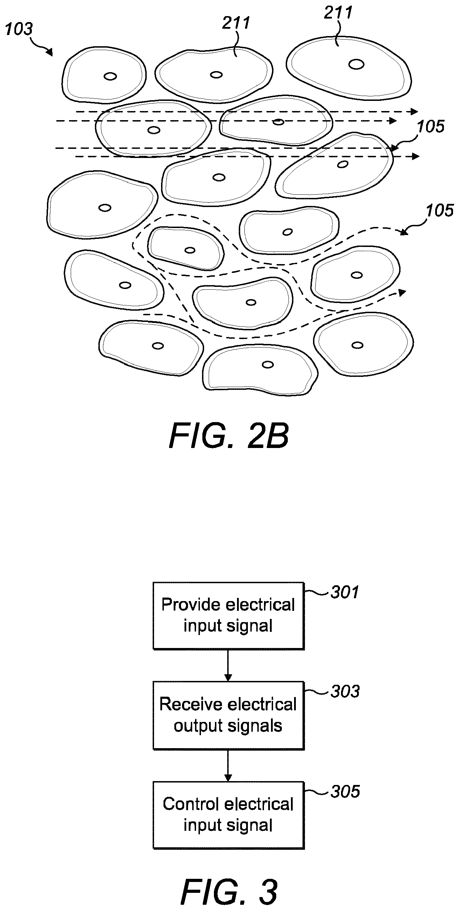

In the example shown in Fig. 2 the example apparatus 101 comprises means 201 for providing an electrical input signal 105 to the biological sample 103 and means 203 for receiving an electrical output signal 107 from the biological sample 103. The apparatus 101 could also comprise means for analysing electrical output signals 107 however this is not shown in Fig. 2A.

The means 201 providing an electrical input signal 105 to the biological sample 103 can comprise two or more electrodes 205. The electrodes 205 can be positioned on the biological sample 103 so as to enable current from the electrodes 205 to flow from a first electrode 205 and through the biological sample 103 to the electrodes 207. In the example of Fig. 2A the electrodes 205 are positioned on the skin of the user.

The apparatus 101 can be configured to enable parameters of the electrical input signal 105 to be adjusted as appropriate. The parameters that could be adjusted could comprise current amplitudes, voltage amplitudes, waveform, frequency or any other suitable parameter.

The control of the electrical input signal 105 can enable control of the interactions of the electrical input signal 105 with electrical properties within the biological sample 103. This can then control the properties of the electrical output signals 107 obtained by the apparatus 101 from the biological sample 103. The electrical input signal 105 can be adjusted to control general features and sub-features within the electrical output signals 107. The electrical input signal 105 can be adjusted to control the size, shape, position and other suitable properties of the general features and sub-features within the electrical output signals 107.

The means for providing the electrical input signal 105 to the biological sample 103 can be configured to enable the electrical input signal 105 to be provided to different parts of the biological sample 103 at different times. For example, the means for providing the electrical input signal 105 to the biological sample 103 could comprise a plurality of pairs of electrodes 205. The plurality of pairs of electrodes 205 could be arranged in different positions on the biological sample 103. The different pairs of the electrodes 205 could then be configured to provide the electrical input signal 105 to different parts of the biological sample 103 at different times.

The example apparatus 101 also comprises means 203 for receiving electrical output signals 107 from the biological sample 103. In this example the means 203 comprises a plurality of electrodes 207 that can be positioned on the biological sample 103. The electrodes 207 can be positioned on the biological sample 103 so as to enable an electrical output signal 107 to be received at one or more respective electrodes 207. The electrodes 207 can be arranged so that the received electrical output signal 107 flows through a part of the biological sample 103 that interacted with the electrical input signal 105.

In some examples the means 201 for providing a plurality of electrical input signals 105 and means for receiving a plurality of electrical output signals 107 could be configured to enable the plurality of electrical output signals 107 to be detected from different parts of the biological sample 103 at different times. For example, the means 201 for providing the plurality of electrical input signals 105 and the means 203 for receiving a plurality of electrical output signals 107 could comprise a plurality of pairs of electrodes 205, 207. The plurality of pairs of electrodes 205, 207could be provided in different positions on the biological sample 103. The different pairs of the electrodes 205, 207 could then be configured to provide a plurality of electrical input signals 105 to, and receive a plurality of electrical output signals 107 from, different parts of the biological sample 103 at different times.

In some examples, an electrode 205, 207 can form a stimulation pair with any other electrode 205, 207 for delivering electrical input signal to the biological sample 103, and all remaining electrodes 205, 207 can form pairs of any combination to collect electrical output signals 107.

The use of the different electrical input signals 105 at different parts of the biological sample 103 can enable characteristics of the biological sample 103 to be tracked both in time and in space. This can enable characteristics of different parts of the biological sample 103 to be identified. This can also enable changes of the characteristics to be identified. This can be used to detect and/or monitor movement of the biological sample 103 and/or other types of changes.

Fig. 2A also shows a section of a biological sample 103. In this example the biological sample 103 comprises a part of a person. The biological sample 103 comprises a user's skin. In the example of Fig. 2A the electrodes 205, 207 of the apparatus 101 are positioned on the skin.

As shown in Fig. 2A the biological sample 103 comprises a plurality of different types of tissue 209. The different types of tissue can have different electrical responses to the electrical input signals 105. The different tissues can also comprise different structures within them. For example, the different tissues in the biological sample 103 shown in Fig. 2A can comprise capillaries, arterioles, arteries and other structures. Other types of structures could be provided within the biological sample 103 in other examples of the disclosure. For instance, the biological sample could comprise bones or muscles or any other types of structures or other components.

Characteristics of these tissues 209 such as their thickness or shape or the presence of other components within the tissues 209 can be detected using the apparatus 101. In some examples the apparatus 101 can also be configured to detect movement or other changes in these tissues 209.

Fig. 2B shows a section of a biological sample 103 in more detail. This shows some of the cells 211 within the biological sample 103. Fig. 2B shows that an electrical input signal 105 can be provided so that it passes through the cells 211 or so that it passes around the cells 211. Whether the electrical input signal 105 passes through the cells 211 or passes around the cells 211 can determine how the electrical input signal 105 interacts with the electrical properties of the biological sample 103. This can affect the values in the electrical output signals 107 that are detected by the apparatus 101. For example, this can affect any general features and the sub-features within the electrical output signals 107. This could change the amplitude, shape or other properties of the general features and the sub-features within the electrical output signals 107. The way in which the general features and the sub-features within the electrical output signals 107 are affected can be used to identify one or more characteristics of the biological sample 103.

Fig. 3 shows a method that can be implemented using an apparatus 101 as shown in Figs. 1 and 2. The method enables information to be obtained from a biological sample 103 such that the information can be used to identify characteristics of the biological sample 103.

The method comprises, at block 301, providing an electrical input signal 105 to a biological sample 103. The electrical input signal 105 interacts with the biological sample 103 so as to elicit one or more electrical properties of the biological sample 103. The electrical properties of the biological sample 103 that are elicited could comprise any one or more of electrical responses such as resistance, capacitance, impedance, dielectric, phase or any other suitable property.

The electrical input signals 105 can be provided using any suitable means. In some examples the electrical input signals 105 can be provided by two or more electrodes 205 positioned on the biological sample 103. The two or more electrodes 205 can be positioned in any suitable locations on the biological sample 103. In some examples the electrodes 205 can be positioned adjacent to each other. In some examples the electrodes 205 can be positioned on different sides of the biological sample 103 to each other.

In some examples the electrodes 205 can be configured to provide a plurality of pairs of electrodes 205. The plurality of different pairs can comprise any suitable combinations of electrodes 205 within the available set of electrodes 205. The plurality of different pairs of electrodes 205 can be configured so that the electrical input signals 105 can be provided by different pairs of the electrodes 205 at different times. This can enable the electrical input signals 105 to be provided to different parts of the biological sample 103 at different times.

At block 303 the method also comprises receiving a plurality of electrical output signals 107 from the biological sample 103. The electrical output signals 107 correspond to the electrical input signals 105. The electrical output signals 107 correspond to the electrical input signals 105 in that they are provided by the electrical input signals 105 passing through the biological sample 103. As the received electrical output signals 107 have passed through the biological sample 103 the values of the electrical output signals 107 are determined by the electrical properties of the biological sample 103.

The electrical output signals 107 can be received using any suitable means. In some examples the electrical output signals 107 can be received by one or more electrodes 207 positioned on the biological sample 103. The one or more electrodes 207 can be positioned in any suitable locations on the biological sample 103. The electrodes 207 that are used to receive the electrical output signals 107 can be positioned so that the electrical input signal 105 has passed through a part of the biological sample 103 before it is received by the electrode 207.

The method also comprises, at block 305, controlling the electrical input signals 105 so that the plurality of electrical output signals 107 from the biological sample 103 comprise one or more general features and one or more sub-features. The values of the electrical output signals 107 can be determined by the electrical properties of the biological sample 103 and how these interact with the electrical input signals 105. Therefore, by controlling the electrical input signal 105 the values of the electrical output signals 107 can be tuned so as to provide one or more general features and one or more sub-features in the electrical output signals 107.

The one or more general features and the one or more sub-features enable one or more characteristics of the biological sample 103 to be identified. The one or more general features and the one or more sub-features can comprise measurement points within one or more of the electrical output signals 107.

The general features and the sub-features in the electrical output signals 107 can comprise measurement points that enable different types of biological samples 103 to be differentiated from each other. For example, a first pattern of general features and the sub-features can indicate that the biological sample 103 is a first type of sample and a second pattern of general features and sub-features can indicate that the biological sample 103 is a second type of sample.

The sub-features can comprise features that are more subtle than the general features. For instance, in some examples the sub-features can comprise features that can be identified by comparison with reference signals whereas the general features could comprise features that can be identified without comparison to reference signals.

In some examples the general features can comprise one or more global extrema within one or more of the electrical output signals 107 and the sub-features can comprise local extrema within the one or more electrical output signals 107. For instance, the general features could comprise global maxima or minima within the electrical output signals 107 and the sub-features could comprise local maxima or minima. The local maxima or minima could have a smaller amplitude than the global maxima or minima.

In some examples the one or more general features can comprise a most significant feature within the electrical output signals 107 and the one or more sub-features can comprise less significant features within the electrical output signals 107. The sub-features can be less significant in that they are more hidden and less obvious or visible within the electrical output signals 107. The sub-features could have smaller amplitudes or be provided closer to other sub-features within the output signal.

Different electrical output signals 107 can comprise different general features and sub-features. For instance, in some examples, the biological sample 103 can be probed in a plurality of different directions by applying the electrical input signal 105 and the electrical output signal 107 through different pairs of electrodes in different directions across the biological sample 103. The different electrical output signals 107 could then comprise information obtained from the different directions.

In some examples changes of the biological sample 103 could also enable the biological sample 103 to be probed in different directions. The changes of the biological sample 103 could comprise movement of the biological sample 103, movement of parts of the biological sample 103 or any other suitable changes of the biological sample 103.

The measurement points that provide the general features and sub-features can be obtained by probing the biological sample 103 using one or more different temporal patterns. The different temporal patterns can comprise different time points, intervals, cycles, periodicities or any other parameters for the electrical input signals 105.

The controlling of the electrical input signals 105 can comprise controlling one or more parameters so as to create and tune the one or more general features and one or more sub-features in the electrical output signal 107. The parameters of the electrical input signal 105 that can be controlled can comprise current amplitudes, voltage amplitudes, waveform, frequency or any other suitable parameter.

In some examples the input electrical input signal 105 can be controlled to enable different general features and/or sub-features to be provided in the electrical output signals 107. For instance, changing one or more parameters in the input electrical input signal 105 can cause a corresponding change in the general features and/or sub-features in the output signals 107. These changes in the general features and/or sub-features can be used to identify characteristics of the biological sample 103.

In some examples the characteristics of the biological sample 103 can be identified by correlating the one or more general features and one or more sub-features in the electrical output signals 107 from the biological sample 103 with one or more reference signals. The reference signals could be obtained through in-vivo and in-vitro measurements or from simulations or by any other suitable means. If there is a sufficient level of correlation with a reference signal, then this can provide an identification of one or more characteristics of the biological sample 103.

In some examples the method can comprise comparing at least some of the features in the electrical output signals 107 with experimentally determined features to enable changes within the biological sample 103 to be identified.

Some examples of characteristics of the biological sample 103 that can be identified can comprise movement of the biological sample 103, position of the biological sample 103, size of the biological sample 103, shape of the biological sample 103, conductivity of the biological sample 103. In some examples the characteristics can be characteristics of part of the biological sample 103. For example, the characteristics could be the size and/or shape of a type of tissue within the biological sample 103 or of any other suitable part of the biological sample 103. Other characteristics could be used in other examples of the disclosure.

In some examples the apparatus 101 can be configured to perform additional steps in order to enable the characteristics of the biological sample 103 to be identified. For instance, the apparatus 101 can be configured to selectively combine one or more general features and one or more sub-features from one or more electrical output signals 107 and use that combination to identify one or more characteristics of the biological sample 103. In some examples the electrical output signal 107 can be processed to parse the general features and sub-features into different proportions or ratios. The processing that is performed can ensure that there is at least one feature that can be used to identify characteristics of the biological sample 103.

The use of the general features and sub-features within the electrical output signals 107 can enable the characteristics of the biological sample 103 to be identified without generating a reconstruction of the biological sample 103. For instance, there would be no need to generate a reconstruction such as an image or other model of the biological sample 103 in order to enable the characteristics to be identified. This can enable the characteristics to be identified more quickly and accurately and can reduce the processing requirements of the apparatus 101. In some examples this can enable the identification of the characteristics to be used as an input for an electronic device or computer program. For example, it could enable the electrical output signals 107, or data obtained from the electrical output signals 107, to be used to provide a control input to a device or computer program or any other suitable entity.

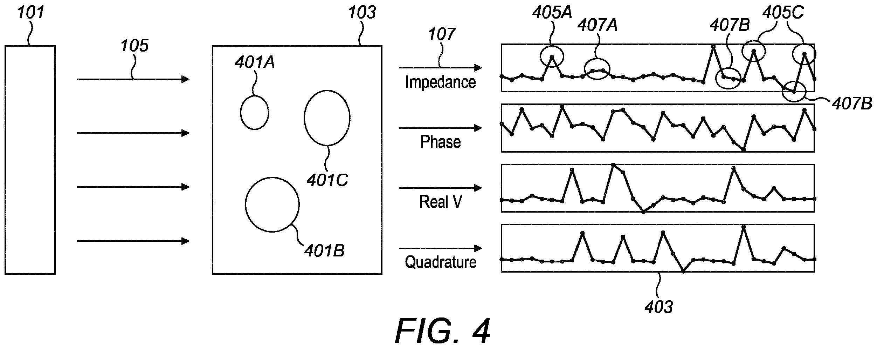

Fig. 4 schematically shows an implementation of examples of the disclosure. In this example the apparatus 101 can be configured to detect movement of the biological sample 103. For instance, the biological sample 103 could comprise part of an arm of a user and the apparatus 101 could be configured to detect movements of the user's muscles when the user makes a gesture using their arm. The gesture could be a two-finger pinch gesture comprising a user bringing two of their fingers together or any other suitable type of gesture.

Fig. 4 shows an apparatus 101 that is configured to provide a plurality of electrical input signals 105 to a biological sample 103.

The parameters of the electrical input signals 105 can be controlled so as to tune electrical output signals 107 to comprise general features and sub-features that can be used to identify characteristics of the biological sample 103.

The parameters of the electrical input signals 105 that are controlled can comprise any suitable parameters and/or combination of parameters. In some examples the parameters could comprise the drive pattern for the electrical input signals 105. The drive pattern could comprise the sequence in which the available electrodes 205 are used to provide the electrical input signals 105. In some examples the parameters could comprise the frequency of the electrical input signals 105. In some examples the parameters could comprise the arrangement of the electrodes 205 that are used to provide the electrical input signals 105. For example, it could comprise the positions of the electrodes 205 on the biological sample 103 and/or the positions of the electrodes 205 relative to each other. Other parameters could be used in other examples of the disclosure.

The electrical input signals 105 are provided to the biological sample 103. In this example the biological sample 103 comprises three muscles 401 that can move as the user makes the gesture. For example, the muscles 401A-C can expand or contract as the user moves their fingers. This can alter the shape of the muscles 401 within the biological sample 103. The muscles 401 are shown schematically in Fig. 4.

The electrical input signals 105 can change the electrical properties of the muscles 401 and/or the parts of the biological sample 103 around the muscles 401. When an electrical input signal 105 is provided to the biological sample 103 this will pass through the muscles 401 to provide the electrical output signals 107. The modification of the electrical properties of the muscles 401 therefore determines the values of the electrical output signals 107.

Fig. 4 also shows a plurality of plots 403 indicative of the electrical output signals 107. The plots 403 shown in Fig. 4 show values of different parameters of the electrical output signals 107. In the example of Fig. 4 the plots show values of impedance, phase, voltage and quadrature. The vertical axis of the plots indicates these values. Other parameters could be used in other examples of the disclosure.

The horizontal axis of these plots 403 corresponds to the different observations or measurements made by the physical electrodes 205, 207. The values are obtained for signals obtained between different pairs of electrodes 205, 207. The different pairs of electrodes 205, 207 can correspond to different parts of the biological sample 103 and/or to different probing directions of the biological sample 103. In this example, each of the plots 403 comprise thirty-two different measurements along the horizontal axis. These measurements are obtained using different pairs of electrodes 205, 207 to provide electrical input signals 105 and electrical output signals 107. Other numbers of measurements could be used in other examples of the disclosure.

The plots 403 comprise general features 405 and sub-features 407. The general features 405 comprise measurement points that can be easily identified within the plots 403. In this example the general features 405 can comprise the global maxima of one or more parameters within the electrical output signals 107. The sub-features 407 can comprise measurement points that have a smaller amplitude compared to the general features. The sub-features 407 could comprise a sequence of measurement points that have a predefined pattern. The sub-features 407 could comprise local maxima or any other suitable type of measurement points.

In the example of Fig. 4 each of the muscles 401 provide different general features 405 and sub-features 407. These general features 405 and sub-features 407 can provide an indication of the shape or position of each of the muscles 401. The combination of these different general features 405 and sub-features 407 can therefore be used to identify patterns of movement of the muscles 401 and so can identify when a gesture has been performed.

In the example of Fig. 4 the first set comprises a general feature 405A and sub-feature 407A. The first set can correspond to a first muscle 401A in that the amplitude and position of these features 405A, 407A are determined by the characteristics of the first muscle 401A. The second set comprises two sub-features 407B. The second set can correspond to a second muscle 401B in that the amplitude and position of these features 407B are determined by the characteristics of the second muscle 401B. The third set comprises two general features 405C. The third set can correspond to a third muscle 401C in that the amplitude and position of these features 405C are determined by the characteristics of the third muscle 401C.

In order to identify whether or not a gesture has been determined the values of the parameters within the electrical output signals 107 can be compared to one or more reference signals. In some examples only a section of the electrical output signals 107 might be compared to the reference signals. For instance, only the sections of the electrical output signals 107 in which the general features 405 and/or the sub-features 407 are expected to be might be compared.

The reference signals used for the comparison can be obtained from experimental results, from simulations or by any other suitable means. The comparison with the reference signals can provide an indication as to whether or not a gesture has been performed. For instance, the electrical output signals 107, or parts of the electrical output signals 107 can be correlated with the reference signals and if the correlation is within a threshold amount it can be determined that the general features 405 and sub-features 407 are indicative of the gesture being performed.

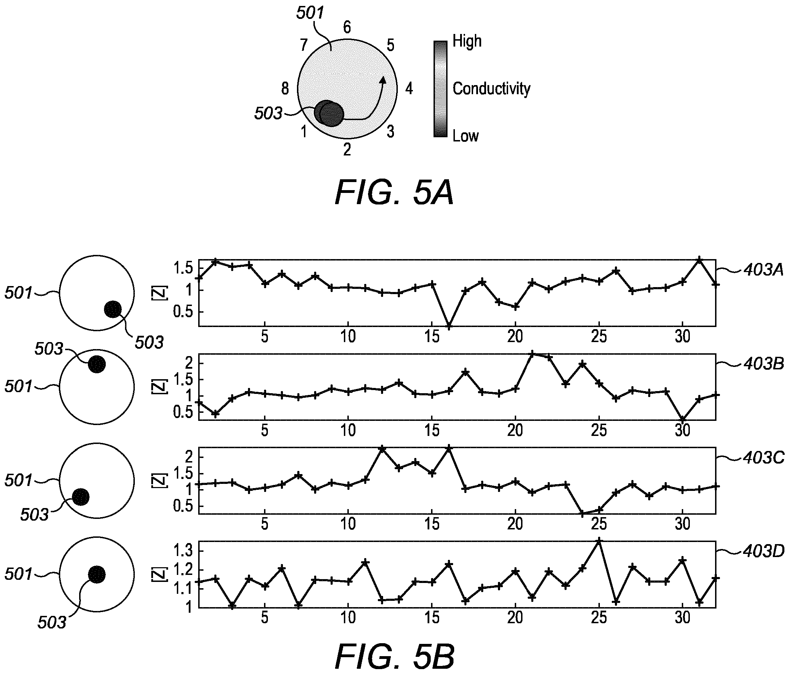

Figs. 5A and 5B shows how examples of the disclosure can be used to detect changes of a biological sample 103.

Fig. 5A schematically shows an object 503 within a circular receptacle 501. The object comprises a wooden cylinder 503. The wooden cylinder 503 can move around within the circular receptacle 501. The wooden cylinder 503 and circular receptacle 501 provide a simplified model of biological sample 103 and are used in this example to show how different electrical output signals 107 are provided when there are changes within the biological sample 103. In this example the changes within a biological sample 103 are represented by changes in position of the wooden cylinder 503.

In order to obtain the electrical output signal 107 a plurality of electrodes is positioned around the edge of the circular receptacle 501. The electrodes can be configured to enable an electrical input signal 105 to be input to the circular receptacle 501 and pass through the circular receptacle 501 and the wooden cylinder 503 to provide an electrical output signal 107.

In this example eight electrodes are distributed around the edge of the circular receptacle 501. The electrodes are provided at regular intervals around the edge of the circular receptacle 501. The circular receptacle 501 can be probed at different directions by using different pairs of the electrodes for the electrical input signal 105 and the electrical output signals 107. Different permutations of the available electrodes can be used to probe the circular receptacle 501 at different directions and at different times.

Fig. 5B shows plots 403A -403D of different electrical output signals 107 that could be obtained for different configurations of the wooden cylinder 503 and circular receptacle 501. The plots correspond to experimental data that was obtained using the wooden cylinder 503 and circular receptacle 501.

The plots 403A- 403D shown in Fig. 5B show values of impedance of the electrical output signals 107. In other examples, the 403A- 403D could show values of other electrical properties such as phase angle, quadrature and or any other suitable parameter. In this example, the vertical axis of the plots 403A- 403D indicates these impedances. The horizontal axis of these plots 403A- 403D correspond to the different permutations of pairs of electrodes that are used. Each of the plots 403A-403D comprise thirty-two different measurements along the horizontal axis. These measurements are obtained using different pairs of electrodes to provide electrical input signals 105 and electrical output signals 107.

In the first plot 403A the wooden cylinder 503 was positioned in a lower right position within the circular receptacle 501. In the second plot 403B the wooden cylinder 503 was positioned in an upper position within the circular receptacle 501. In the third plot 403C the wooden cylinder 503 was positioned in a lower left position within the circular receptacle 501. In the fourth plot 403D the wooden cylinder 503 was positioned in a central position within the circular receptacle 501.

Each of the plots 403A, 403B, 403C, 403D comprise different measure points. The different measurement points provide different general features and sub-features. These general features and sub-features can therefore be used to identify characteristics such as the position or movement of the wooden cylinder 503 within the circular receptacle 501.

Therefore, these experimental results show how different general features and sub-features within an electrical output signal 107 can be used to identify at least the position of an object. This could therefore be used to identify characteristics such as the position of components within a biological sample 103.

The use of different directions to probe the circular receptacle 501 can enable small movements of the wooden cylinder 503 to be detected. The movements of the wooden cylinder 503 can cause a change in different parts of the electrical output signal 107 corresponding to measurements made between different electrodes. This causes a change in the general features and sub-features within the electrical output signal 107 that enables the movements to be identified.

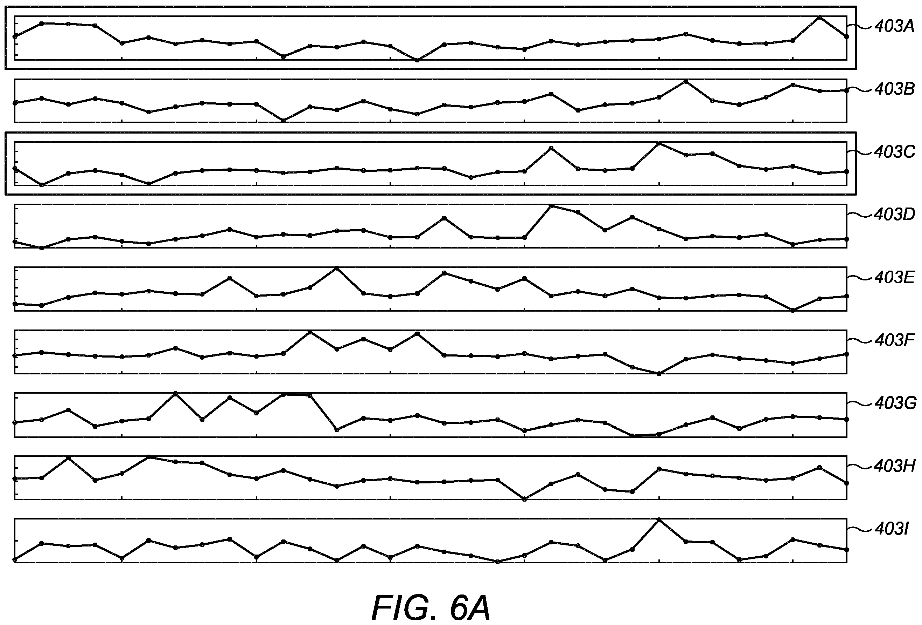

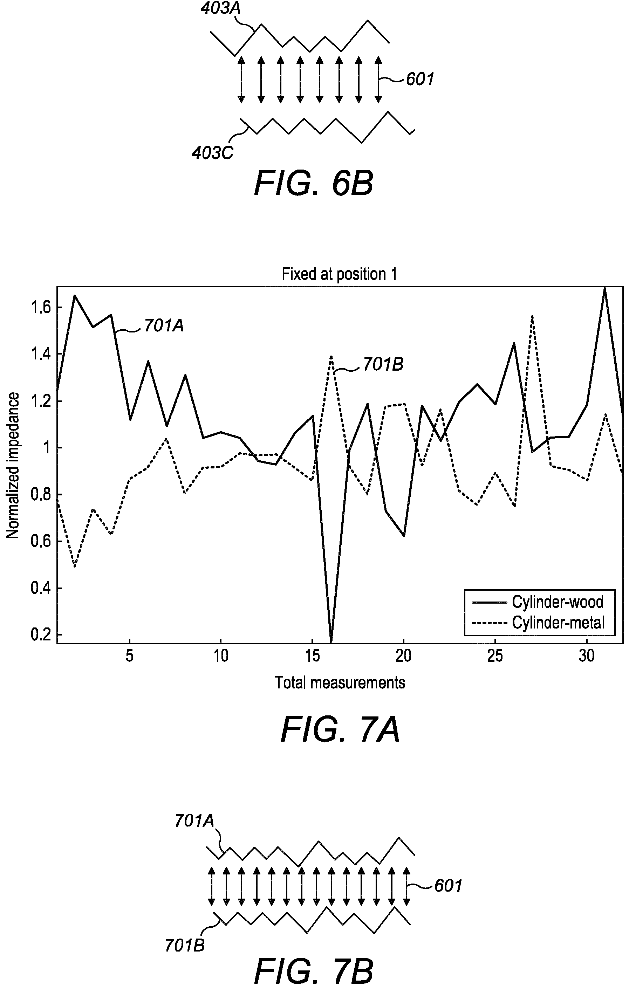

Figs. 6A and 6B show example output signals. Fig. 6A shows a plurality of different plots 403A- 403I corresponding to electrical output signals 107 received at different times. These plots can be obtained from a wooden cylinder 503 moving around a circular receptacle 501 as shown in Fig. 5A.

The plots 403A- 403I shown in Fig. 6A show values of impedance of the electrical output signals 107. The vertical axis of the plots indicates these impedances. The horizontal axis of these plots 403A- 403I corresponds to the different permutations of pairs of electrodes that are used. Each of the plots 403A-403I comprise thirty-two different measurements along the horizontal axis. These measurements are obtained using different pairs of electrodes to provide electrical input signals 105 and electrical output signals 107.

Fig. 6B shows a comparison between two of the plots. In this example the comparison is made between the first plot 403A and the third pot 403C. The comparison could be made between any permutations comprising two or more of the plots.

To make the comparison a plurality of data points from the first plot 403A are compared to a plurality of data points from the second plot 403C. In this example only a subset of the available data points is compared. In other examples all of the available data points from the two plots 403A, 403C could be compared.

In the example of Fig. 6B the data points are compared from the fifteenth data point in each of the plots 403A, 403C. Other sections of the plots 403A, 403C could be used in other examples of the disclosure.

The section of the plots that are to be compared can be selected based on the information that is to be collected. For example, different parts of the plots 403A, 403C can correspond to different parts of the circular receptacle 501. The area of the circular receptacle 501 in which the wooden cylinder 503 is moving through could provide the most useful information as this could be the area that is changing.

The comparisons enable the differences between the two plots to be compared as indicated by the arrows 601. These differences, rather than the original plots 403A, 403B can be used to identify the features and sub-features in the electrical output signals 107.

The examples show in Figs. 6A and 6B have been obtained using a wooden cylinder 503 moving around within a circular receptacle 501 however it is to be appreciated that the principles can be extended to biological samples 103.

Figs. 7A and 7B show example output signals. Fig. 7A shows a first plot 701A and a second plot 701B. The first plot 701A is obtained using a wooden cylinder 503 within a circular receptacle 501 as shown in Fig. 5A. The second plot 701B is obtained by replacing the wooden cylinder 503 with a metal cylinder. As the wooden cylinder 503 and the metal cylinder have different electrical properties, they provide different electrical output signals 107.

The plots 701A and 701B shown in Fig. 7A show values of impedance of the electrical output signals 107. The vertical axis of the plots indicates these impedances. The horizontal axis of these plots 701A and 701B corresponds to the different permutations of pairs of electrodes that are used. Each of the plots 701A and 701B comprise thirty-two different measurements along the horizontal axis. These measurements are obtained using different pairs of electrodes to provide electrical input signals 105 and electrical output signals 107. These plots clearly show different general features and sub-features due to the different electrical properties of the cylinders that have been used.

Fig. 7B shows a comparison between sections of the two plots 701A and 701B. To make the comparison a plurality of data points from the first plot 701A are compared to a plurality of data points from the second plot 701B. In this example only a subset of the available data points is compared. In other examples all of the available data points from the two plots 701A and 701B could be compared.

The comparisons enable the differences between the two plots 701A and 701B to be compared as indicated by the arrows 601. These differences, rather than the original plots 701A and 701B can be used to identify the general features and sub-features in the electrical output signals 107.

The arrows 601 show example comparisons between example parts of the 701A and 701B. Other parts of the plots 701A and 701B could be compared in other examples. The parts of the plots 701A and 701B that are to be compared can be determined through experiments. For instance, it can be identified that a first point indicates responses from an upper part of a muscle while a second point indicates responses from a lower part of a muscle. Comparing these two points can therefore give an indication of the contraction of that muscle.

The examples shown in Figs. 7A and 7B have been obtained using a wooden cylinder and metal cylinder 503 in a static position within a circular receptacle 501 however it is to be appreciated that the principles can be extended to biological samples 103. For example, the electrical signals 105 can be used to change the electrical properties of the biological sample 103 or of a part of the biological sample 103. This can provide different plots that can then be compared as shown in Figs. 7A and 7B so that the characteristics of the biological sample can be identified using the general features and the sub-features.

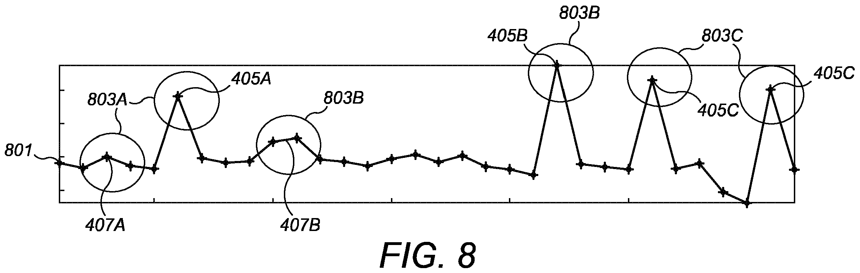

Fig. 8 shows another example plot 801 obtained from electrical output signals 107. The plot 801 could be obtained from a biological sample 103 using methods and apparatus 101 as described herein.

In this example the biological sample 103 can be a complex sample comprising different types of tissues or other entities that can move or that have other properties that can change. For example, if the biological sample 103 comprises part of a user's arm, then different muscles within the arm can expand and contract as the user makes gestures or other movements.

The changes in the different components within the biological sample 103 can result in different patterns being identifiable within the output signals 107. The plot in Fig. 8 shows three different patterns 803A. 803B and 803C. Each of these patterns 803A, 803B and 803C can correspond to different tissues or other components within the biological sample 103.

The different patterns can comprise different general features 405 and sub-features 407. The different general features and sub-features can enable the different characteristics of the biological sample 103 to be identified. In this example the different characteristics can comprise the different muscles and movement or other changes of these muscles.

In this example the first pattern 803A comprises a general feature 405A and a sub-feature 407A. The second pattern 803B comprises a general feature 405B and a sub-feature 407B. The third pattern 803C comprises two general features 405C. Other patterns comprising other arrangements of general features and sub-features 407 can be obtained in other examples of the disclosure.

The expected patterns for a given type of biological sample 103 and/or gesture made using a biological sample 103 could be determined from experimental data. For instance, a plurality of test biological samples 103 comprising known tissues could be used to make some predetermined gestures and the data could be collected to determine a reference output signal comprising expected general features 405 and sub-features 407 for a given type of biological sample 103 and/or gestures. When the apparatus 101 is being used the electrical output signals 107 obtained from the biological sample 103 can be compared to the reference signals to enable characteristics of the biological sample 103 to be identified.

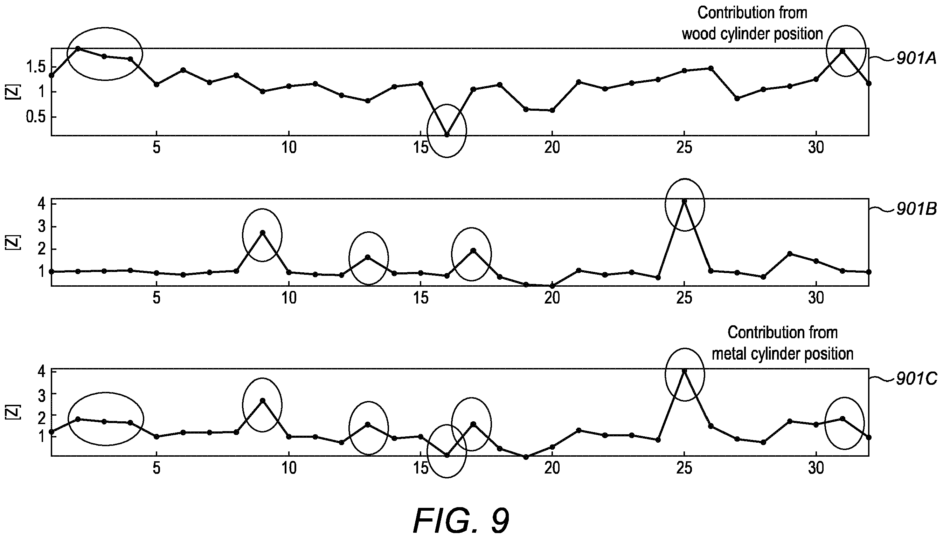

Fig. 9 shows example plots 901A-901C obtained from electrical output signals 107. These plots 901A-901C are obtained using a wooden cylinder 503 and a metal cylinder provided within a circular receptacle 501 as described above. These plots 901A-901C show that patterns resulting from the two different types of cylinder can be identified in the electrical output signals 107.

The first plot 901A is obtained when only a wooden cylinder 503 was located within the circular receptacle 501. This shows a pattern comprising general features and sub-features. In particular, this shows a cluster of sub-features at the left hand side of the plot 901A, a general feature comprising a minima in the centre of the plot 901A and a general feature comprising a maxima at the right hand edge of the plot 901A.

The second plot 901B is obtained when only a metal cylinder was located within the circular receptacle 501. This shows a different pattern comprising different general features and sub-features. In particular this shows a plurality of sub-features around the middle of the plot 901B, and a general feature comprising a maxima positioned towards the right hand side of the plot 901B.

The third plot 901C is obtained when both a wooden cylinder 503 and a metal cylinder were located within the circular receptacle 501. This third plot 901C shows the patterns from both the first plot 901A and the second plot 901B. The general features and the sub-features from both the first plot 901A and the second plot 901B can be identified in the third plot 901C. This indicates that two different types of cylinders were present. By comparing the general features and sub-features in the third plot 901C to the general features and the sub-features in the first plot 901A and the second plot 901B, the type of cylinders that were used, among other characteristics of the cylinders, can be identified.

This demonstrates that patterns corresponding to specific components can be identified within the electrical output signals 107. The identification of these patterns can enable characteristics of complex biological samples 103 to be identified. This shows a validation experiment that shows that a correlation can be established between the general features and the sub-features in different electrical output signal signals 107. When the examples of the disclosure are being used to monitor a biological sample 103 the plots obtained from the biological sample 103 can be compared to one or more reference signals.

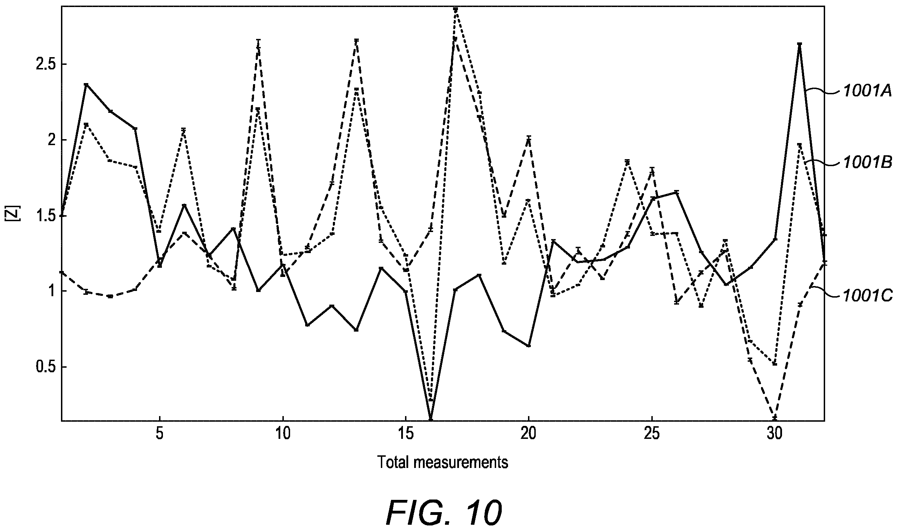

Fig. 10 shows example plots 1001A-1001C obtained from electrical output signals. The example of Fig. 10 shows three different plots. Each of the plots 1001A-1001C comprise thirty-two different measurements obtained using different pairs of electrodes to provide an input signal 105 and an electrical output signal 107.

The data in the different plots 1001A-1001C shown in Fig. 10 were obtained using a wooden cylinder 503 in a circular receptacle 501 as described previously. In this example the wooden cylinder was not moving and remained in the same position within the circular receptacle 501. In this example the three different plots were obtained by using three different simulation conditions. For example, different parameters for the electrical input signals 105 could be used to obtain the three different plots 1001A-1001C.

In the particular example of Fig. 10 the electrical input signals 105 could be provided between two opposing electrodes to obtain a first plot 1001A and between electrodes that are offset diagonally to obtain the other plots 1001B and 1001C. This generates three different electrical output signals 107 comprising general features and sub-features that are unique to the sample object or type of object being sampled.

The plots shown in Fig. 10 are obtained from a model that is used to represent a biological sample 103. This shows that the general features and sub-features of the electrical output signals 107 can be modified by controlling the parameters of the electrical input signals 105 and can therefore be used to identify characteristics of the biological sample 103.

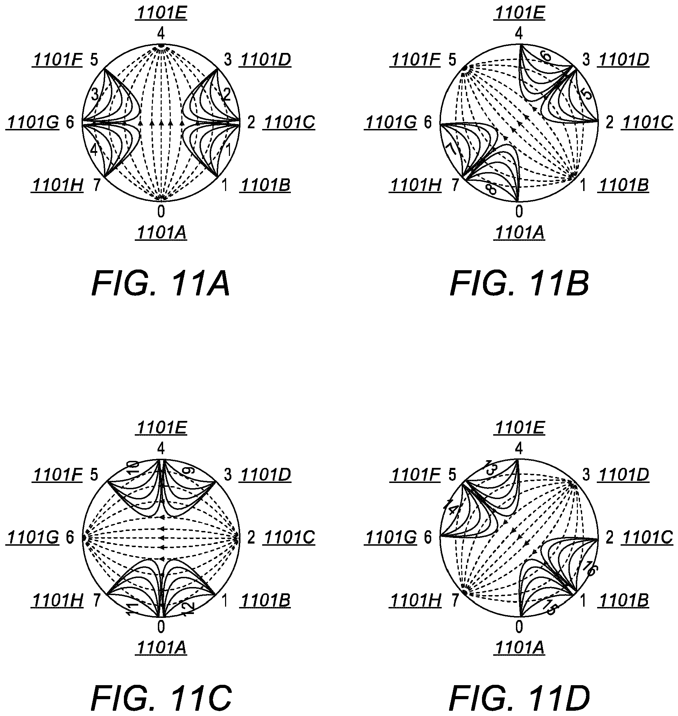

Figs. 11A to 11H schematically show different example of current injection and measurement pairings that can be used to obtain the different measurements.

This example shows eight different electrodes 1101A-1101H that could be positioned on a biological sample 103. In this example, eight different electrodes 1101A-1101H are arranged around the circumference of a circle. The eight different electrodes 1101A-1101H are arranged at equal angular intervals around the circumference of a circle. Other arrangements for the electrodes 1101A-1101H can be used in other examples of the disclosure.

In this example the electrodes 1101A-1101H are in fixed positions. The electrodes 1101A-1101H in this example do not move relative to the biological sample 103.

In this example the electrodes 1101A-1101H are configured so that they can be used for providing measurement signals, that is, the electrical input signals 105 and the electrical output signals 107. The electrodes 1101A-1101H can be used to provide different signals at different times.

In these examples each of the electrodes 1101A-1101H can form a stimulation pair with any other electrode 1101A-1101H for delivering electrical input signals 105 to the biological sample 103. The remaining electrodes 1101A-1101H can form pairs of any combination to collect electrical output signals 107.

In these examples the dashed lines show the electrical input signals 105 and the solid lines show the electrical output signals 107.

In Fig. 11A the electrical input signals 105 are provided from electrode 1101A to electrode 1101E. Electrical output signals 107 signals are received by the pairs of electrodes that are not used to provide the electrical input signals 105. In the example of Fig. 11A, four sets of electrical output signals 107 are provided. These are provided between electrodes 1101B and 1101C, between electrodes 1101C and 1101D, between electrodes 1101F and 1101G, and between electrodes 1101G and 1101H. This provides four data points for the plots as shown herein.

Figs. 11B to 11H show similar arrangements for the electrical input signals 105 and the electrical output signals 107. However, by changing the electrodes that are used the biological sample 103 can be probed in different directions and/or the measurement signals can be obtained from different areas.

In Fig. 11B the electrical input signals 105 are provided from electrode 1101B to electrode 1101F. The electrical output signals 107 are received between electrodes 1101C and 1101D, between electrodes 1101D and 1101E, between electrodes 1101G and 1101H, and between electrodes 1101H and 1101A.

In Fig. 11C the electrical input signals 105 are provided from electrode 1101C to electrode 1101G. The electrical output signals 107 are received between electrodes 1101D and 1101E, between electrodes 1101E and 1101F, between electrodes 1101H and 1101A, and between electrodes 1101A and 1101B.

In Fig. 11D the electrical input signals 105 are provided from electrode 1101D to electrode 1101H. The electrical output signals 107 are received between electrodes 1101E and 1101F, between electrodes 1101F and 1101G, between electrodes 1101A and 1101B, and between electrodes 1101B and 1101C.

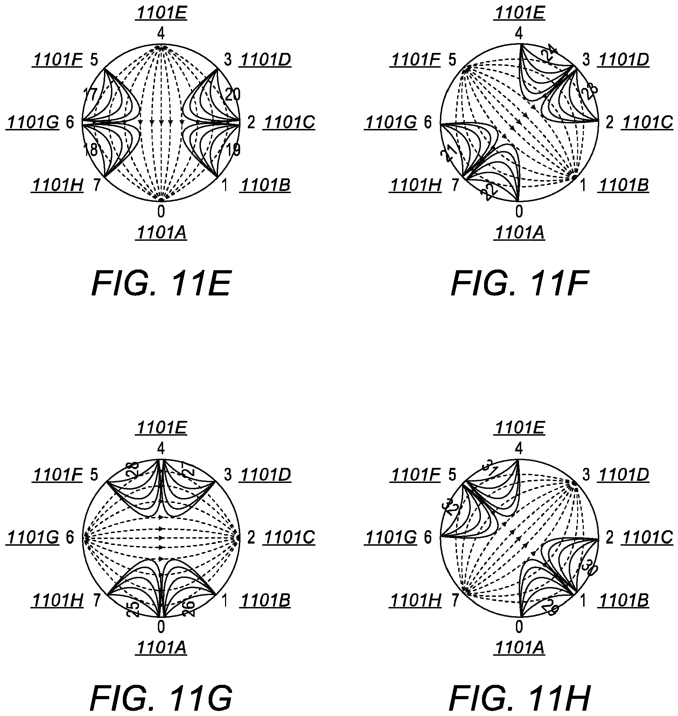

In Fig. 11E the electrical input signals 105 are provided from electrode 1101E to electrode 1101A. The electrical output signals 107 are received between electrodes 1101F and 1101G, between electrodes 1101G and 1101H, between electrodes 1101B and 1101C, and between electrodes 1101C and 1101D.

In Fig. 11F the electrical input signals 105 are provided from electrode 1101F to electrode 1101B. The electrical output signals 107 are received between electrodes 1101G and 1101H, between electrodes 1101H and 1101A, between electrodes 1101C and 1101D, and between electrodes 1101D and 1101E.

In Fig. 11G the electrical input signals 105 are provided from electrode 1101G to electrode 1101C. The electrical output signals 107 are provided between electrodes 1101H and 1101A, between electrodes 1101A and 1101B, between electrodes 1101D and 1101E, and between electrodes 1101E and 1101F.

In Fig. 11H the electrical input signals 105 are provided from electrode 1101H to electrode 1101D. The electrical output signals 107 are provided between electrodes 1101A and 1101B, between electrodes 1101B and 1101C, between electrodes 1101E and 1101F, and between electrodes 1101F and 1101G.

Therefore in total this provides thirty-two data points for the plots as shown herein. This shows that a large number of data points can be obtained from just eight electrodes. Other numbers, arrangements, and/or configurations of electrodes could be used in other examples of the disclosure. If a larger number of electrodes are used then this could enable an even larger number of data points to be obtained. This can increase the resolution of the electrical output signal 107 that is obtained for identification of characteristics of biological samples 103.

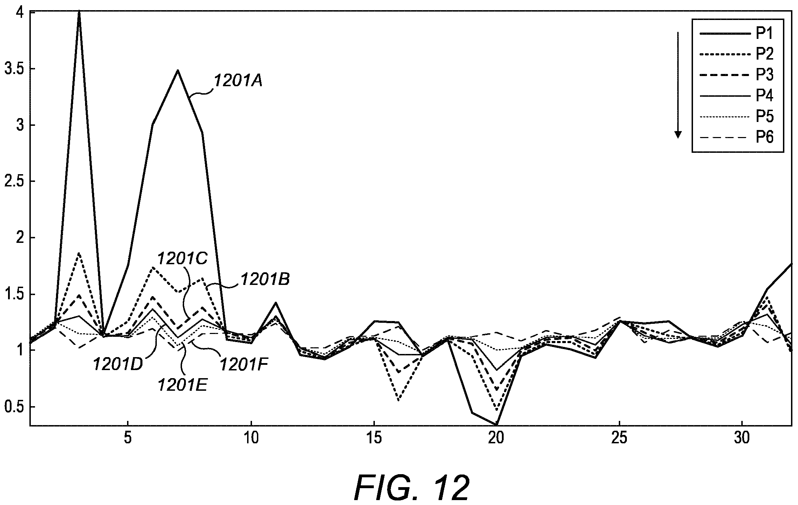

Fig. 12 shows example plots 1201A-1201F obtained from electrical output signals. The example of Fig. 12 shows six different plots. Each of the plots 1201A-1201F comprise thirty-two different measurements obtained using different pairs of electrodes such as the electrode pairs shown in Fig. 11.

In this example the different plots are provided by movement of an object within a biological sample 103. For example, a muscle could move within a biological sample 103. The different plots 1201A-1201F can be obtained at different times as the parts of the biological sample 103 are moving. The different plots obtained at different times could be used to identify the movement of the muscle. The movement of the muscle could provide an indication of a type of gesture that is being performed.

In some examples electrical output signals 107 and/or the different plots obtained from the electrical output signals 107 can be combined so as to provide a multi-dimensional data set.

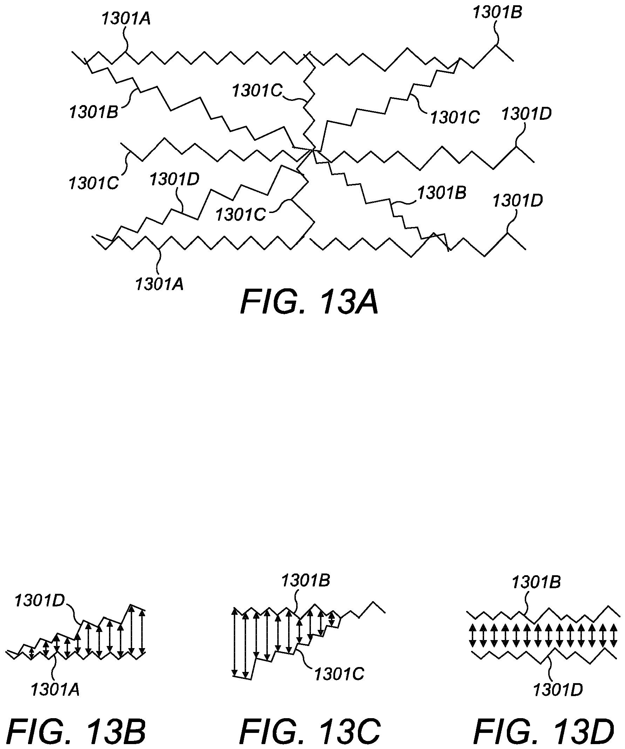

Figs. 13A to 13D shows example plots 1301A-1301D obtained from electrical output signals 107. The plots 1301A-1301D can be compared and/or combined with each other to enable to provide multi-dimensional datasets from which general features and/or sub-features can be identified.

The combining and/or mixing of the different plots 1301A-1301D is possible because each of the points or measurements in 1301A-1301D is independent of the other points or measurements in 1301A-1301D. This can also enable different parts of the plots 1301A-1301D to be aligned with any other part of the other plots 1301A-1301D. For example, sections of a first plot can be combined and/or mixed with sections of another plot so as to enable general features and/or sub-features to be identified.

Fig. 13A shows a plurality of different plots. In this example four different plots 1301A-1301D are shown. The different plots 1301A-1301D are shown in some different combinations to show how they could be combined.

Fig. 13B shows a comparison of two plots 1301A and 1301D. The differences between them are indicated by the arrows. The differences between the two plots 1301A and 1301D provide an indication of a general feature and/or sub-features of the electrical output signals 107. Fig. 13C shows a comparison between another two different plots 1301C and 1301B. The differences between these plots 1301C and 1301B can provide an indication of another general feature and/or sub-features. Fig. 13D shows a comparison between a further set of two different plots 1301D and 1301B. The differences between these plots 1301D and 1301B can provide an indication of a further general feature and/or sub-features.

Therefore, by combining and/or comparing different plots, different features and/or sub-features can be identified. This can enable more accurate identification of the characteristics of the biological sample 103 and/or could enable different types of biological sample to be identified.

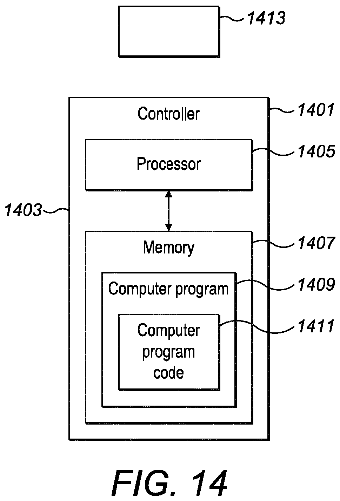

Fig. 14 schematically illustrates a controller apparatus 1401 according to examples of the disclosure. The controller apparatus 1401 illustrated in Fig. 14 can be a chip or a chip-set. In some examples the controller apparatus 1401 can be provided within a computer or other device that be configured to provide and receive signals.

In the example of Fig. 14 the controller apparatus 1401 comprises a controller 1403. In the example of Fig. 14 the implementation of the controller 1403 can be as controller circuitry. In some examples the controller 1403 can be implemented in hardware alone, have certain aspects in software including firmware alone or can be a combination of hardware and software (including firmware).

As illustrated in Fig. 14 the controller 1403 can be implemented using instructions that enable hardware functionality, for example, by using executable instructions of a computer program 1409 in a general-purpose or special-purpose processor 1405 that can be stored on a computer readable storage medium (disk, memory etc.) to be executed by such a processor 1405.

The processor 1405 is configured to read from and write to the memory 1407. The processor 1405 can also comprise an output interface via which data and/or commands are output by the processor 1405 and an input interface via which data and/or commands are input to the processor 1405.

The memory 1407 is configured to store a computer program 1409 comprising computer program instructions (computer program code 1411) that controls the operation of the controller apparatus 1401 when loaded into the processor 1405. The computer program instructions, of the computer program 1409, provide the logic and routines that enables the controller apparatus 1401 to perform the methods illustrated in Fig. 3 The processor 1405 by reading the memory 1407 is able to load and execute the computer program 1409.

The controller apparatus 1401 therefore comprises: at least one processor 1405; and at least one memory 1407 including computer program code 1411, the at least one memory 1407 and the computer program code 1411 configured to, with the at least one processor 1405, cause the controller apparatus 1401 at least to perform:
providing a plurality of electrical input signals 105 to a biological sample 103;
receiving a plurality of electrical output signals 107 from the biological sample 103 where the electrical output signals 107 correspond to the electrical input signals 105 and the values of the electrical output signals 107 are based on one or more electrical properties of the biological sample 103; and
controlling the electrical input signals 105 so that the plurality of electrical output signals 107 from the biological sample 103 comprise one or more general features and one or more sub-features where the one or more general features and the one or more sub-features enable one or more characteristics of the biological sample 103 to be identified.

As illustrated in Fig. 14 the computer program 1409 can arrive at the controller apparatus 1401 via any suitable delivery mechanism 1413. The delivery mechanism 1413 can be, for example, a machine readable medium, a computer-readable medium, a non-transitory computer-readable storage medium, a computer program product, a memory device, a record medium such as a Compact Disc Read-Only Memory (CD-ROM) or a Digital Versatile Disc (DVD) or a solid state memory, an article of manufacture that comprises or tangibly embodies the computer program 1409. The delivery mechanism can be a signal configured to reliably transfer the computer program 1409. The controller apparatus 1401 can propagate or transmit the computer program 1409 as a computer data signal. In some examples the computer program 1409 can be transmitted to the controller apparatus 1401 using a wireless protocol such as Bluetooth, Bluetooth Low Energy, Bluetooth Smart, 6LoWPan (IPᵥ6 over low power personal area networks) ZigBee, ANT+, near field communication (NFC), Radio frequency identification, wireless local area network (wireless LAN) or any other suitable protocol.

The computer program 1409 comprises computer program instructions for causing a controller apparatus 1401 to perform at least the following:
providing a plurality of electrical input signals 105 to a biological sample 103;
receiving a plurality of electrical output signals 107 from the biological sample 103 where the electrical output signals 107 correspond to the electrical input signals 105 and the values of the electrical output signals 107 are based on one or more electrical properties of the biological sample 103; and
controlling the electrical input signals 105 so that the plurality of electrical output signals 107 from the biological sample 103 comprise one or more general features and one or more sub-features where the one or more general features and the one or more sub-features enable one or more characteristics of the biological sample 103 to be identified.

The computer program instructions can be comprised in a computer program 1409, a non-transitory computer readable medium, a computer program product, a machine readable medium. In some but not necessarily all examples, the computer program instructions can be distributed over more than one computer program 1409.

Although the memory 1407 is illustrated as a single component/circuitry it can be implemented as one or more separate components/circuitry some or all of which can be integrated/removable and/or can provide permanent/semi-permanent/ dynamic/cached storage.

Although the processor 1405 is illustrated as a single component/circuitry it can be implemented as one or more separate components/circuitry some or all of which can be integrated/removable. The processor 1405 can be a single core or multi-core processor.

References to "computer-readable storage medium", "computer program product", "tangibly embodied computer program" etc. or a "controller", "computer", "processor" etc. should be understood to encompass not only computers having different architectures such as single /multi- processor architectures and sequential (Von Neumann)/parallel architectures but also specialized circuits such as field-programmable gate arrays (FPGA), application specific circuits (ASIC), signal processing devices and other processing circuitry. References to computer program, instructions, code etc. should be understood to encompass software for a programmable processor or firmware such as, for example, the programmable content of a hardware device whether instructions for a processor, or configuration settings for a fixed-function device, gate array or programmable logic device etc.

As used in this application, the term "circuitry" can refer to one or more or all of the following:
(a) hardware-only circuitry implementations (such as implementations in only analog and/or digital circuitry) and
(b) combinations of hardware circuits and software, such as (as applicable):
   (i) a combination of analog and/or digital hardware circuit(s) with software/firmware and
   (ii) any portions of hardware processor(s) with software (including digital signal processor(s)), software, and memory(ies) that work together to cause an apparatus, such as a mobile phone or server, to perform various functions and
(c) hardware circuit(s) and or processor(s), such as a microprocessor(s) or a portion of a microprocessor(s), that requires software (e.g. firmware) for operation, but the software can not be present when it is not needed for operation.

This definition of circuitry applies to all uses of this term in this application, including in any claims. As a further example, as used in this application, the term circuitry also covers an implementation of merely a hardware circuit or processor and its (or their) accompanying software and/or firmware. The term circuitry also covers, for example and if applicable to the particular claim element, a baseband integrated circuit for a mobile device or a similar integrated circuit in a server, a cellular network device, or other computing or network device.

The blocks illustrated in Fig. 3 can represent steps in a method and/or sections of code in the computer program 1409. The illustration of a particular order to the blocks does not necessarily imply that there is a required or preferred order for the blocks and the order and arrangement of the blocks can be varied. Furthermore, it can be possible for some blocks to be omitted.

The systems, apparatus 101, methods and computer programs 1409 can use machine learning which can include statistical learning. Machine learning is a field of computer science that gives computers the ability to learn without being explicitly programmed. The computer learns from experience E with respect to some class of tasks T and performance measure P if its performance at tasks in T, as measured by P, improves with experience E. The computer can often learn from prior training data to make predictions on future data. Machine learning includes wholly or partially supervised learning and wholly or partially unsupervised learning. It may enable discrete outputs (for example classification, clustering) and continuous outputs (for example regression). Machine learning may for example be implemented using different approaches such as cost function minimization, artificial neural networks, support vector machines and Bayesian networks for example. Cost function minimization may, for example, be used in linear and polynomial regression and K-means clustering. Artificial neural networks, for example with one or more hidden layers, model complex relationship between input vectors and output vectors. Support vector machines can be used for supervised learning. A Bayesian network is a directed acyclic graph that represents the conditional independence of a number of random variables.

The term 'comprise' is used in this document with an inclusive not an exclusive meaning. That is any reference to X comprising Y indicates that X may comprise only one Y or may comprise more than one Y. If it is intended to use 'comprise' with an exclusive meaning then it will be made clear in the context by referring to "comprising only one..." or by using "consisting".

In this description, reference has been made to various examples. The description of features or functions in relation to an example indicates that those features or functions are present in that example. The use of the term 'example' or 'for example' or 'can' or 'may' in the text denotes, whether explicitly stated or not, that such features or functions are present in at least the described example, whether described as an example or not, and that they can be, but are not necessarily, present in some of or all other examples. Thus 'example', 'for example', 'can' or 'may' refers to a particular instance in a class of examples. A property of the instance can be a property of only that instance or a property of the class or a property of a sub-class of the class that includes some but not all of the instances in the class. It is therefore implicitly disclosed that a feature described with reference to one example but not with reference to another example, can where possible be used in that other example as part of a working combination but does not necessarily have to be used in that other example.

Although examples have been described in the preceding paragraphs with reference to various examples, it should be appreciated that modifications to the examples given can be made without departing from the scope of the claims.

Features described in the preceding description may be used in combinations other than the combinations explicitly described above.

Although functions have been described with reference to certain features, those functions may be performable by other features whether described or not.

Although features have been described with reference to certain examples, those features may also be present in other examples whether described or not.

The term 'a' or 'the' is used in this document with an inclusive not an exclusive meaning. That is any reference to X comprising a/the Y indicates that X may comprise only one Y or may comprise more than one Y unless the context clearly indicates the contrary. If it is intended to use 'a' or 'the' with an exclusive meaning then it will be made clear in the context. In some circumstances the use of 'at least one' or 'one or more' may be used to emphasis an inclusive meaning but the absence of these terms should not be taken to infer any exclusive meaning.

The presence of a feature (or combination of features) in a claim is a reference to that feature or (combination of features) itself and also to features that achieve substantially the same technical effect (equivalent features). The equivalent features include, for example, features that are variants and achieve substantially the same result in substantially the same way. The equivalent features include, for example, features that perform substantially the same function, in substantially the same way to achieve substantially the same result.

In this description, reference has been made to various examples using adjectives or adjectival phrases to describe characteristics of the examples. Such a description of a characteristic in relation to an example indicates that the characteristic is present in some examples exactly as described and is present in other examples substantially as described.

Whilst endeavoring in the foregoing specification to draw attention to those features believed to be of importance it should be understood that the Applicant may seek protection via the claims in respect of any patentable feature or combination of features hereinbefore referred to and/or shown in the drawings whether or not emphasis has been placed thereon.

## Claims

1. An apparatus (101) comprising means for:
providing (301) a plurality of electrical input signals (105) to a biological sample (103);
receiving (303) a plurality of electrical output signals (107) from the biological sample (103) where the electrical output signals (107) correspond to the electrical input signals (105) and the values of the electrical output signals (107) are based on one or more electrical properties of the biological sample (103);
controlling (305) the electrical input signals (105) so that the plurality of electrical output signals (107) from the biological sample (103) comprise one or more general features and one or more sub-features where the one or more general features and the one or more sub-features enable one or more characteristics of the biological sample (103) to be identified;
wherein the one or more general features and the one or more sub-features comprise measurement points within the plurality of electrical output signals (107); and
wherein the one or more general features comprise one or more global minima or maxima within one or more of the electrical output signals (107) and the one or more sub-features comprise local minima or maxima within the one or more electrical output signals (107).

2. An apparatus as claimed in any previous claim wherein the one or more general features and the one or more sub-features comprise one or more measurement points that are obtained by probing the biological sample in a plurality of different directions.

3. An apparatus as claimed in claim 2 wherein the biological sample can be probed in different directions by at least one of: providing the electrical input signals in different directions across the biological sample, receiving the electrical output signals from different directions across the biological sample, changes of the biological sample.

4. An apparatus as claimed in any previous claim wherein the one or more general features and the one or more sub-features comprise one or more measurement points that are obtained by probing the biological sample using one or more different temporal patterns, wherein the different temporal patterns comprise different: time points, intervals, cycles, or periodicities.

5. An apparatus as claimed in any preceding claim wherein the means are for selectively combining one or more general features and one or more sub-features from one or more of the electrical output signals to identify one or more characteristics of the biological sample.

6. An apparatus as claimed in any preceding claim wherein the one or more general features and one or more sub-features in the electrical output signals are configured to enable one or more characteristics of the biological sample to be identified without generating at least one of: an image of the biological sample; a model of the biological sample.

7. An apparatus as claimed in any preceding claim wherein controlling the electrical input signals comprises controlling one or more parameters of the electrical input signals so as to create and tune the one or more general features and one or more sub-features in the electrical output signals.

8. An apparatus as claimed in any preceding claim wherein the means for providing the electrical input signals to the biological sample are configured to enable the electrical input signals to be provided to different parts of the biological sample at different times and/ or the means for receiving a plurality of electrical output signals are configured to enable the plurality of electrical output signals to be detected from different parts of the biological sample at different times.

9. An apparatus as claimed in any preceding claim wherein the one or more characteristics of the biological sample are identified by correlating the one or more general features and one or more sub-features in the electrical output signals from the biological sample with one or more reference signals.

10. An apparatus as claimed in any preceding claim wherein the means are for comparing at least some of the features in the electrical output signals with experimentally determined features to enable changes within the biological sample to be identified.

11. An apparatus as claimed in any preceding claim wherein the electrical output signals are used to provide a control input to one or more of: a device, a computer program.

12. A method comprising:
providing (301) a plurality of electrical input signals (105) to a biological sample (103);
receiving (303) a plurality of electrical output signals (107) from the biological sample (103) where the electrical output signals (107) correspond to the electrical input signals (105) and the values of the electrical output signals (107) are based on one or more electrical properties of the biological sample (103);
controlling (305) the electrical input signals (105) so that the plurality of electrical output signals (107) from the biological sample (103) comprise one or more general features and one or more sub-features where the one or more general features and the one or more sub-features enable one or more characteristics of the biological sample (103) to be identified;
wherein the one or more general features and the one or more sub-features comprise measurement points within the plurality of electrical output signals (107); and
wherein the one or more general features comprise one or more global minima or maxima within one or more of the electrical output signals (107) and the one or more sub-features comprise local minima or maxima within the one or more electrical output signals (107).

13. A computer program (1409) comprising computer program instructions (1411) that, when executed by processing circuitry, cause the apparatus (101) of claim 1 to carry out the steps of:
providing (301) a plurality of electrical input signals (105) to a biological sample (103);
receiving (303) a plurality of electrical output signals (107) from the biological sample (103) where the electrical output signals (107) correspond to the electrical input signals (105) and the values of the electrical output signals (107) are based on one or more electrical properties of the biological sample (103);
controlling (305) the electrical input signals (105) so that the plurality of electrical output signals (107) from the biological sample (103) comprise one or more general features and one or more sub-features where the one or more general features and the one or more sub-features enable one or more characteristics of the biological sample (103) to be identified;
wherein the one or more general features and the one or more sub-features comprise measurement points within the plurality of electrical output signals (107); and
wherein the one or more general features comprise one or more global minima or maxima within one or more of the electrical output signals (107) and the one or more sub-features comprise local minima or maxima within the one or more electrical output signals (107).

## Patentansprüche

1. Einrichtung (101), die Mittel für Folgendes umfasst:
Bereitstellen (301) einer Vielzahl von elektrischen Eingangssignalen (105) für eine biologische Probe (103);
Empfangen (303) einer Vielzahl von elektrischen Ausgangssignalen (107) von der biologischen Probe (103), wo die elektrischen Ausgangssignale (107) den elektrischen Eingangssignalen (105) entsprechen und die Werte der elektrischen Ausgangssignale (107) auf einer oder mehreren elektrischen Eigenschaften der biologischen Probe (103) basieren;
Steuern (305) der elektrischen Eingangssignale (105), derart, dass die Vielzahl von elektrischen Ausgangssignalen (107) von der biologischen Probe (103) ein oder mehrere allgemeine Merkmale und ein oder mehrere Untermerkmale umfassen, wo das eine oder die mehreren allgemeinen Merkmale und das eine oder die mehreren Untermerkmalen es ermöglichen, ein oder mehrere Charakteristika der biologischen Probe (103) zu identifizieren;
wobei das eine oder die mehreren allgemeinen Merkmale und das eine oder die mehreren Untermerkmalen Messpunkte in der Vielzahl von elektrischen Ausgangssignalen (107) umfassen; und
wobei das eine oder die mehreren allgemeinen Merkmale ein oder mehrere globale Minima oder Maxima in einem oder mehreren der elektrischen Ausgangssignale (107) umfassen und das eine oder die mehreren Untermerkmalen lokale Minima oder Maxima in dem einen oder den mehreren elektrischen Ausgangssignalen (107) umfassen.

2. Einrichtung nach einem der vorhergehenden Ansprüche, wobei das eine oder die mehreren allgemeinen Merkmale und das eine oder die mehreren Untermerkmale ein oder mehrere Messpunkte umfassen, die durch Sondieren der biologischen Probe in einer Vielzahl von verschiedenen Richtungen erhalten werden.

3. Einrichtung nach Anspruch 2, wobei die biologische Probe in verschiedenen Richtungen durch mindestens eines von Folgendem sondiert werden kann: Bereitstellen der elektrischen Eingangssignale in verschiedenen Richtungen über die biologische Probe, Empfangen der elektrischen Ausgangssignale aus verschiedenen Richtungen über die biologische Probe, Ändern der biologischen Probe.

4. Einrichtung nach einem der vorhergehenden Ansprüche, wobei das eine oder die mehreren allgemeinen Merkmale und das eine oder die mehreren Untermerkmale einen oder mehrere Messpunkte umfassen, die durch Sondieren der biologischen Probe unter Verwendung von einem oder mehreren verschiedenen Zeitmustern erhalten werden, wobei die verschiedenen Zeitmuster verschiedene Zeitpunkte, Intervalle, Zyklen oder Periodizitäten umfassen.

5. Einrichtung nach einem der vorhergehenden Ansprüche, wobei die Mittel dem selektiven Kombinieren von einem oder mehreren allgemeinen Merkmalen und einem oder mehreren Untermerkmalen von einem oder mehreren der elektrischen Ausgangssignale dienen, um ein oder mehrere Charakteristika der biologischen Probe zu identifizieren.

6. Einrichtung nach einem der vorhergehenden Ansprüche, wobei das eine oder die mehreren allgemeinen Merkmale und das eine oder die mehreren Untermerkmale in den elektrischen Ausgangssignalen dazu ausgelegt sind, es zu ermöglichen, dass ein oder mehrere Charakteristika der biologischen Probe identifiziert werden, ohne mindestens eines von Folgendem zu erzeugen: einem Bild der biologischen Probe; einem Modell der biologischen Probe.

7. Einrichtung nach einem der vorhergehenden Ansprüche, wobei das Steuern der elektrischen Eingangssignale das Steuern von einem oder mehreren Parametern der elektrischen Eingangssignale umfasst, um das eine oder die mehreren allgemeinen Merkmale und das eine oder die mehreren Untermerkmale in den elektrischen Ausgangssignalen zu erstellen und abzustimmen.

8. Einrichtung nach einem der vorhergehenden Ansprüche, wobei die Mittel zum Bereitstellen der elektrischen Eingangssignale für die biologische Probe dazu ausgelegt sind, es zu ermöglichen, dass die elektrischen Eingangssignale verschiedenen Teilen der biologischen Probe zu verschiedenen Zeiten bereitgestellt werden, und/oder die Mittel zum Empfangen einer Vielzahl von elektrischen Ausgangssignalen dazu ausgelegt sind, es zu ermöglichen, die Vielzahl von elektrischen Ausgangssignalen von verschiedenen Teilen der biologischen Probe zu verschiedenen Zeiten zu detektieren.

9. Einrichtung nach einem der vorhergehenden Ansprüche, wobei das eine oder die mehreren Charakteristika der biologischen Probe durch Korrelieren des einen oder der mehreren allgemeinen Merkmale und des einen oder der mehreren Untermerkmale in den elektrischen Ausgangssignalen von der biologischen Probe mit einem oder mehreren Referenzsignalen identifiziert werden.

10. Einrichtung nach einem der vorhergehenden Ansprüche, wobei die Mittel dem Vergleichen von mindestens einigen der Merkmale in den elektrischen Ausgangssignalen mit experimentell bestimmten Merkmalen dienen, um es zu ermöglichen, Änderungen in der biologischen Probe zu identifizieren.

11. Einrichtung nach einem der vorhergehenden Ansprüche, wobei die elektrischen Ausgangssignale verwendet werden, um einem oder mehrerem von Folgendem eine Steuereingabe bereitzustellen: einer Vorrichtung, einem Computerprogramm.

12. Verfahren, das Folgendes umfasst:
Bereitstellen (301) einer Vielzahl von elektrischen Eingangssignalen (105) für eine biologische Probe (103);
Empfangen (303) einer Vielzahl von elektrischen Ausgangssignalen (107) von der biologischen Probe (103), wo die elektrischen Ausgangssignale (107) den elektrischen Eingangssignalen (105) entsprechen und die Werte der elektrischen Ausgangssignale (107) auf einer oder mehreren elektrischen Eigenschaften der biologischen Probe (103) basieren;
Steuern (305) der elektrischen Eingangssignale (105), derart, dass die Vielzahl von elektrischen Ausgangssignalen (107) von der biologischen Probe (103) ein oder mehrere allgemeine Merkmale und ein oder mehrere Untermerkmale umfassen, wo das eine oder die mehreren allgemeinen Merkmale und das eine oder die mehreren Untermerkmalen es ermöglichen, ein oder mehrere Charakteristika der biologischen Probe (103) zu identifizieren;
wobei das eine oder die mehreren allgemeinen Merkmale und das eine oder die mehreren Untermerkmalen Messpunkte in der Vielzahl von elektrischen Ausgangssignalen (107) umfassen; und
wobei das eine oder die mehreren allgemeinen Merkmale ein oder mehrere globale Minima oder Maxima in einem oder mehreren der elektrischen Ausgangssignale (107) umfassen und das eine oder die mehreren Untermerkmalen lokale Minima oder Maxima in dem einen oder den mehreren elektrischen Ausgangssignalen (107) umfassen.

13. Computerprogramm (1409), das Computerprogrammanweisungen (1411) umfasst, die, wenn sie von einer Verarbeitungsschaltung ausgeführt werden, die Einrichtung (101) von Anspruch 1 veranlassen, die folgenden Schritte umzusetzen:
Bereitstellen (301) einer Vielzahl von elektrischen Eingangssignalen (105) für eine biologische Probe (103);
Empfangen (303) einer Vielzahl von elektrischen Ausgangssignalen (107) von der biologischen Probe (103), wo die elektrischen Ausgangssignale (107) den elektrischen Eingangssignalen (105) entsprechen und die Werte der elektrischen Ausgangssignale (107) auf einer oder mehreren elektrischen Eigenschaften der biologischen Probe (103) basieren;
Steuern (305) der elektrischen Eingangssignale (105), derart, dass die Vielzahl von elektrischen Ausgangssignalen (107) von der biologischen Probe (103) ein oder mehrere allgemeine Merkmale und ein oder mehrere Untermerkmale umfassen, wo das eine oder die mehreren allgemeinen Merkmale und das eine oder die mehreren Untermerkmalen es ermöglichen, ein oder mehrere Charakteristika der biologischen Probe (103) zu identifizieren;
wobei das eine oder die mehreren allgemeinen Merkmale und das eine oder die mehreren Untermerkmalen Messpunkte in der Vielzahl von elektrischen Ausgangssignalen (107) umfassen; und
wobei das eine oder die mehreren allgemeinen Merkmale ein oder mehrere globale Minima oder Maxima in einem oder mehreren der elektrischen Ausgangssignale (107) umfassen und das eine oder die mehreren Untermerkmalen lokale Minima oder Maxima in dem einen oder den mehreren elektrischen Ausgangssignalen (107) umfassen.

## Revendications

1. Appareil (101) comprenant des moyens pour :
fournir (301) une pluralité de signaux d'entrée électriques (105) à un échantillon biologique (103) ;
recevoir (303) une pluralité de signaux de sortie électriques (107) de l'échantillon biologique (103), où les signaux de sortie électriques (107) correspondent aux signaux d'entrée électriques (105) et les valeurs des signaux de sortie électriques (107) sont basées sur une ou plusieurs propriétés électriques de l'échantillon biologique (103) ;
commander (305) les signaux d'entrée électriques (105) de sorte que la pluralité de signaux de sortie électriques (107) de l'échantillon biologique (103) comprennent une ou plusieurs caractéristiques générales et une ou plusieurs sous-caractéristiques, où les une ou plusieurs caractéristiques générales et les une ou plusieurs sous-caractéristiques permettent d'identifier une ou plusieurs caractéristiques de l'échantillon biologique (103) ;
dans lequel les une ou plusieurs caractéristiques générales et les une ou plusieurs sous-caractéristiques comprennent des points de mesure dans la pluralité de signaux de sortie électriques (107) ; et
dans lequel les une ou plusieurs caractéristiques générales comprennent un ou plusieurs minima ou maxima globaux dans un ou plusieurs des signaux de sortie électriques (107), et les une ou plusieurs sous-caractéristiques comprennent des minima ou maxima locaux dans les un ou plusieurs signaux de sortie électriques (107).

2. Appareil selon l'une des revendications précédentes, dans lequel les une ou plusieurs caractéristiques générales et les une ou plusieurs sous-caractéristiques comprennent un ou plusieurs points de mesure qui sont obtenus en sondant l'échantillon biologique dans une pluralité de directions différentes.

3. Appareil selon la revendication 2, dans lequel l'échantillon biologique peut être sondé dans différentes directions par au moins l'une des actions suivantes : la fourniture des signaux d'entrée électriques dans différentes directions à travers l'échantillon biologique, la réception des signaux de sortie électriques en provenance de différentes directions à travers l'échantillon biologique, des changements de l'échantillon biologique.

4. Appareil selon l'une des revendications précédentes, dans lequel les une ou plusieurs caractéristiques générales et les une ou plusieurs sous-caractéristiques comprennent un ou plusieurs points de mesure qui sont obtenus en sondant l'échantillon biologique en utilisant un ou plusieurs motifs temporels différents, dans lequel les motifs temporels différents comprennent : différents points, intervalles, cycles ou périodicités temporels.

5. Appareil selon l'une des revendications précédentes, dans lequel les moyens sont destinés à combiner de manière sélective une ou plusieurs caractéristiques générales et une ou plusieurs sous-caractéristiques d'un ou plusieurs des signaux de sortie électriques afin d'identifier une ou plusieurs caractéristiques de l'échantillon biologique.

6. Appareil selon l'une des revendications précédentes, dans lequel les une ou plusieurs caractéristiques générales et une ou plusieurs sous-caractéristiques dans les signaux de sortie électriques sont configurés pour permettre d'identifier une ou plusieurs caractéristiques de l'échantillon biologique sans générer au moins un parmi : une image de l'échantillon biologique ; un modèle de l'échantillon biologique.

7. Appareil selon l'une des revendications précédentes, dans lequel la commande des signaux d'entrée électriques comprend la commande d'un ou plusieurs paramètres des signaux d'entrée électriques de manière à créer et à accorder les une ou plusieurs caractéristiques générales et les une ou plusieurs sous-caractéristiques dans les signaux de sortie électriques.

8. Appareil selon l'une des revendications précédentes, dans lequel les moyens pour fournir les signaux d'entrée électriques à l'échantillon biologique sont configurés pour permettre de fournir les signaux d'entrée électriques à différentes parties de l'échantillon biologique à différents moments, et/ou les moyens pour recevoir une pluralité de signaux de sortie électriques sont configurés pour permettre de détecter la pluralité de signaux de sortie électriques à partir de différentes parties de l'échantillon biologique à différents moments.

9. Appareil selon l'une des revendications précédentes, dans lequel les une ou plusieurs caractéristiques de l'échantillon biologique sont identifiées en corrélant les une ou plusieurs caractéristiques générales et les une ou plusieurs sous-caractéristiques dans les signaux de sortie électriques de l'échantillon biologique avec un ou plusieurs signaux de référence.

10. Appareil selon l'une des revendications précédentes, dans lequel les moyens sont destinés à comparer au moins certaines des caractéristiques dans les signaux de sortie électriques avec des caractéristiques déterminées expérimentalement pour permettre d'identifier des changements dans l'échantillon biologique.

11. Appareil selon l'une des revendications précédentes, dans lequel les signaux de sortie électriques sont utilisés pour fournir une entrée de commande à un ou plusieurs parmi : un dispositif, un programme informatique.

12. Procédé comprenant les étapes suivantes :
fournir (301) une pluralité de signaux d'entrée électriques (105) à un échantillon biologique (103) ;
recevoir (303) une pluralité de signaux de sortie électriques (107) de l'échantillon biologique (103), où les signaux de sortie électriques (107) correspondent aux signaux d'entrée électriques (105) et les valeurs des signaux de sortie électriques (107) sont basées sur une ou plusieurs propriétés électriques de l'échantillon biologique (103) ;
commander (305) les signaux d'entrée électriques (105) de sorte que la pluralité de signaux de sortie électriques (107) de l'échantillon biologique (103) comprennent une ou plusieurs caractéristiques générales et une ou plusieurs sous-caractéristiques, où les une ou plusieurs caractéristiques générales et les une ou plusieurs sous-caractéristiques permettent d'identifier une ou plusieurs caractéristiques de l'échantillon biologique (103) ;
dans lequel les une ou plusieurs caractéristiques générales et les une ou plusieurs sous-caractéristiques comprennent des points de mesure dans la pluralité de signaux de sortie électriques (107) ; et
dans lequel les une ou plusieurs caractéristiques générales comprennent un ou plusieurs minima ou maxima globaux dans un ou plusieurs des signaux de sortie électriques (107), et les une ou plusieurs sous-caractéristiques comprennent des minima ou maxima locaux dans les un ou plusieurs signaux de sortie électriques (107).

13. Programme informatique (1409) comprenant des instructions de programme informatique (1411) qui, lorsqu'elles sont exécutées par un ensemble de circuits de traitement, amènent l'appareil (101) de la revendication 1 à exécuter les étapes suivantes :
fournir (301) une pluralité de signaux d'entrée électriques (105) à un échantillon biologique (103) ;
recevoir (303) une pluralité de signaux de sortie électriques (107) de l'échantillon biologique (103), où les signaux de sortie électriques (107) correspondent aux signaux d'entrée électriques (105) et les valeurs des signaux de sortie électriques (107) sont basées sur une ou plusieurs propriétés électriques de l'échantillon biologique (103) ;
commander (305) les signaux d'entrée électriques (105) de sorte que la pluralité de signaux de sortie électriques (107) de l'échantillon biologique (103) comprennent une ou plusieurs caractéristiques générales et une ou plusieurs sous-caractéristiques, où les une ou plusieurs caractéristiques générales et les une ou plusieurs sous-caractéristiques permettent d'identifier une ou plusieurs caractéristiques de l'échantillon biologique (103) ;
dans lequel les une ou plusieurs caractéristiques générales et les une ou plusieurs sous-caractéristiques comprennent des points de mesure dans la pluralité de signaux de sortie électriques (107) ; et
dans lequel les une ou plusieurs caractéristiques générales comprennent un ou plusieurs minima ou maxima globaux dans un ou plusieurs des signaux de sortie électriques (107), et les une ou plusieurs sous-caractéristiques comprennent des minima ou maxima locaux dans les un ou plusieurs signaux de sortie électriques (107).
